(19)

**Europäisches Patentamt**
**European Patent Office**
**Office européen des brevets**

(11)  **EP 4 559 892 A1**

(12)  **EUROPEAN PATENT APPLICATION**
published in accordance with Art. 153(4) EPC

(43) Date of publication:
**28.05.2025 Bulletin 2025/22**

(21) Application number: **23842902.1**

(22) Date of filing: **13.07.2023**

(51) International Patent Classification (IPC):
$C07C\ 31/20^{(2006.01)}$    $C08G\ 18/42^{(2006.01)}$
$C08G\ 63/12^{(2006.01)}$    $C12P\ 7/18^{(2006.01)}$

(52) Cooperative Patent Classification (CPC):
**C07C 31/20; C08G 18/42; C08G 63/12; C12P 7/18**

(86) International application number:
**PCT/JP2023/025825**

(87) International publication number:
**WO 2024/018979 (25.01.2024 Gazette 2024/04)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:  **22.07.2022  JP 2022117126**

(71) Applicant: **DIC Corporation**
**Tokyo 174-8520 (JP)**

(72) Inventors:
• **SATO Kouji**
  **Takaishi-shi, Osaka 592-0001 (JP)**
• **TSUTSUMI Hiroshi**
  **Takaishi-shi, Osaka 592-0001 (JP)**
• **KITADA Mitsuru**
  **Takaishi-shi, Osaka 592-0001 (JP)**
• **MIYAMOTO Masanori**
  **Sakura-shi, Chiba 285-8668 (JP)**
• **SHIGEHIRO Tatsuya**
  **Sakura-shi, Chiba 285-8668 (JP)**

(74) Representative: **TBK**
**Bavariaring 4-6**
**80336 München (DE)**

(54)  **1,6-HEXANEDIOL COMPOSITION AND POLYMER**

(57)  [Problem] An object of the present invention is to provide a 1,6-hexanediol composition that can improve the hydrolysis resistance of a polymer obtained using a 1,6-hexanediol composition as a reaction raw material, and a polymer obtained by reacting the 1,6-hexanediol composition.
[Means for Resolution] The invention relates to a 1,6-hexanediol composition containing either or both of 1,6-hexanediol and a 1,6-hexanediol derivative, and an alkali metal element, in which the total content of the alkali metal element with respect to the total amount of the composition is in the range of 0.1 to 1000 ppm by mass, and the composition has an acid value in the range of 0.001 to 1.0 mg KOH/g.

**EP 4 559 892 A1**

**Description**

TECHNICAL FIELD

The present invention relates to a 1,6-hexanediol composition and a polymer.

BACKGROUND ART

A 1,6-hexanediol (1,6-HD) composition is an intermediate product useful for the production of polymers such as polyesters and polyurethanes. A 1,6-hexanediol composition can be generally produced by (1) hydrogenation of adipic acid or an adipic acid-containing feed flow, (2) hydrogenation of hydroxycaproic acid or an ester thereof, or (3) hydrogenation of caprolactone.

Polymers such as polyesters and polyurethanes obtained by reacting a 1,6-hexanediol composition are widely used in artificial leathers and the like. With regard to polyurethanes, for example, PTL 1 discloses that a polyurethane resin composition including a polyester polyol produced using a polyisocyanate, a polyhydric alcohol, and a polyvalent carboxylic acid in the presence of a solid acid catalyst, has excellent hydrolysis resistance; however, it is necessary to use a special solid catalyst during the production of a polyester polyol, and to recover the solid catalyst, and another technology needs to be provided.

CITATION LIST

PATENT LITERATURE

PTL 1: International Publication No 2010/035579

SUMMARY OF INVENTION

TECHNICAL PROBLEM

It is an object of the present invention is to provide, as the other technology, a 1,6-hexanediol composition that can improve the hydrolysis resistance of a polymer produced using a 1,6-hexanediol composition as a reaction raw material, and a polymer that uses the 1,6-hexanediol composition as a reaction raw material.

SOLUTION TO PROBLEM

The inventors of the present invention conducted intensive studies, and as a result, the inventors found that a polymer obtained by reacting a 1,6-hexanediol composition that contains a specific amount of an alkali metal element and has a specific acid value, has satisfactory hydrolysis resistance, thus completing the present invention.

That is, the present invention provides the following inventions.

[1] A 1,6-hexanediol composition containing either or both of 1,6-hexanediol and a 1,6-hexanediol derivative, and an alkali metal element, wherein a total content of the alkali metal element with respect to a total amount of the composition is in a range of 0.1 to 1000 ppm by mass, and the composition has an acid value in a range of 0.001 to 1.0 mg KOH/g.

[2] The 1,6-hexanediol composition according to [1], wherein the 1,6-hexanediol composition further contains an organic acid, and the organic acid has a pKa value at 25°C of 4.0 or greater.

[3] The 1,6-hexanediol composition according to [1] or [2], wherein the 1,6-hexanediol composition is induced from a biomass resource.

[4] A polymer produced using the 1,6-hexanediol composition according to any one of [1] to [3] as a reaction raw material.

[5] The polymer according to [4], wherein the polymer is a polyester or a polyurethane.

ADVANTAGEOUS EFFECTS OF INVENTION

The 1,6-hexanediol composition of the present invention has an acid value of 0.001 to 1.0 mg KOH/g and a total content of an alkali metal element of 0.1 to 1000 ppm by mass, and thus a polymer produced using the 1,6-hexanediol composition as a reaction raw material has satisfactory hydrolysis resistance.

DESCRIPTION OF EMBODIMENTS

<1,6-Hexanediol composition>

The 1,6-hexanediol composition (hereinafter, simply referred to as composition) of the present invention contains either or both of 1,6-hexanediol and a 1,6-hexanediol derivative, and an alkali metal element, the total content of the alkali metal element with respect to the total amount of the composition is in the range of 0.1 to 1000 ppm by mass, and the acid value of the composition is in the range of 0.001 to 1.0 mg KOH/g.

<<1,6-Hexanediol and 1,6-hexanediol derivative>>

The 1,6-hexanediol included in the composition of the present invention is preferably unmodified 1,6-hexanediol; however, a 1,6-hexanediol derivative may also be used. That is, the composition of the present invention contains at least any one of 1,6-hexanediol and a 1,6-hexanediol derivative.

The 1,6-hexanediol derivative included in the composition of the present invention is a compound in which any one or both of the two hydroxyl groups of the 1,6-hexanediol included in the 1,6-hexanediol composition of the present invention have been modified. Here, as a method for modifying the hydroxyl groups of 1,6-hexanediol, any known method for modifying a hydroxyl group can be used, and examples thereof include an etherification reaction, an esterification reaction, and modification by (meth)acrylic acid.

Examples of the 1,6-hexanediol derivative include epoxy group-containing 1,6-hexanediol derivatives such as 1,6-hexanediol diglycidyl ether and 6-hydroxyhexyl glycidyl ether; (meth)acryloyl group-containing 1,6-hexanediol derivatives such as 1,6-hexanediol di(meth)acrylate, 6-hydroxyhexyl acrylate, and 1,6-hexanediol monoacrylate monomethacrylate; vinyl ether group-containing 1,6-hexanediol derivatives such as 1,6-hexanediol divinyl ether and 1,6-hexanediol monovinyl ether; aliphatic alkyl ether group-containing 1,6-hexanediol derivatives such as 6-propyloxy-1-hexanol, 1,6-dipropoxy-hexane, 1,6-hexanediol methyl ether, and 1,6-dimethoxyhexane; and fatty acid ester group-containing 1,6-hexanediol derivatives such as propyl 6-hydroxyhexanoate, di-n-propyl adipate, methyl 6-hydroxycaproate, and dimethyl adipate. These may be used singly, or two or more kinds thereof may be used in combination.

In the present specification, the term (meth)acryloyl group means either or both of an acryloyl group and a methacryloyl group, and the term (meth)acrylate means either or both of acrylate and methacrylate.

The content of either or both of 1,6-hexanediol and the 1,6-hexanediol derivative in the 1,6-hexanediol composition of the present invention is preferably 96.00% to 99.99% by mass, more preferably 99.00% to 99.99% by mass, and even more preferably 99.50% to 99.99% by mass. By setting the purity of the 1,6-hexanediol composition to be within the above-described range while setting the total content of the alkali metal element to be within the above-described range and setting the acid value of the composition to be within the above-described range, the effects of the present invention tend to be more favorably obtained.

In the present specification, the content of 1,6-hexanediol is a value measured by gas chromatography mass spectrometry (GC-MS).

<<Alkali metal element>>

The composition of the present invention contains an alkali metal element, and the total content of the alkali metal element with respect to the total amount of the composition is 0.1 to 1000 ppm by mass.

The alkali metal element is not particularly limited, and examples thereof include lithium, sodium, potassium, rubidium, and cesium. These may be used singly, or two or more kinds thereof may be used in combination. Among them, sodium and potassium are preferred.

The state of existence of the alkali metal element included in the composition of the present invention is not particularly limited, and may be a simple substance of an alkali metal, an alkali metal compound, an alkali metal ion, or the like.

Specific examples of the alkali metal compound include metal salts of the above-described alkali metal elements and acids such as acetic acid, phosphoric acid, and nitric acid.

With regard to the 1,6-hexanediol composition of the present invention, the upper limit of the total content of the alkali metal element (preferably, the total content of either or both of sodium metal element and potassium metal element) is preferably 1000 ppm by mass or less. The lower limit of the total content is not particularly limited, and is preferably 0.1 ppm by mass or more, more preferably 1 ppm by mass or more, even more preferably 100 ppm by mass or more, and particularly preferably 500 ppm by mass or more. These upper limits and lower limits can be used in any combination.

The total content of the alkali metal element (preferably, the total content of either or both of sodium metal element and potassium metal element) in the 1,6-hexanediol composition of the present invention is 0.1 to 1000 ppm by mass, but is preferably 1 to 1000 ppm by mass, more preferably 100 to 1000 ppm by mass, and even more preferably 500 to 1000 ppm by mass. In the present specification, the total content of the alkali metal element is a value measured by inductively coupled plasma mass spectrometry (ICP-MS).

<<Acid value of 1,6-hexanediol composition of present invention>>

With regard to the acid value of the 1,6-hexanediol composition of the present invention, the upper limit is preferably 1.0 mg KOH/g or less, more preferably 0.8 mg KOH/g or less, and even more preferably 0.6 mg KOH/g or less. The lower limit of the acid value is not particularly limited, and is preferably 0.001 mg KOH/g or greater, more preferably 0.01 mg KOH/g or greater, and even more preferably 0.1 mg KOH/g or greater. These upper limits and lower limits can be used in any combination.

The acid value of the 1,6-hexanediol composition of the present invention is 0.001 to 1.0 mg KOH/g, but is preferably 0.01 to 0.8 mg KOH/g, and more preferably 0.1 to 0.6 mg KOH/g.

In a case where the acid value is greater than the above-described upper limit, when a polymer (for example, a polyester) is produced using the 1,6-hexanediol composition, there is a risk that the polymer may not exhibit the performance of the designed polymer (for example, a polyester). Furthermore, in a case where the acid value is smaller than the above-described lower limit, when a polymer (for example, a polyester or a polyurethane) is synthesized using the 1,6-hexanediol composition, there is a risk that reactivity may decrease. When the acid value is within the range, acceleration of a hydrolysis reaction of the polymer (for example, a polyester) can be favorably suppressed.

In the present specification, the acid value is a value measured according to JIS K 0070-1992.

<<Organic acid>>

It is preferable that the 1,6-hexanediol composition of the present invention further contains an organic acid having a pKa value at 25°C of 4.0 or greater. This tends to allow the effects of the present invention to be obtained more favorably.

The organic acid is not particularly limited as long as the pKa value at 25°C is 4.0 or greater, and examples thereof include acetic acid (pKa value 4.56), succinic acid (pKa value 4.00), propionic acid (pKa value 4.67), adipic acid (pKa value 4.26), valeric acid (pKa value 4.64), pivalic acid (pKa value 5.03), catechol (pKa value 9.23), and phenol (pKa value 9.82). These may be used singly, or two or more kinds thereof may be used in combination. Among them, acetic acid and succinic acid are preferred, and acetic acid is more preferred.

Incidentally, there are compounds exhibiting two or more pKa values in organic acids; however, in the present invention, the pKa value of a compound in that case is the lowest value.

The pKa value at 25°C of the organic acid is 4.0 or greater; however, the pKa value is preferably 4.0 to 10.0, more preferably 4.0 to 8.0, even more preferably 4.0 to 6.0, and particularly preferably 4.0 to 5.0. This tends to allow the effects to be obtained more favorably. In the present specification, the pKa value at 25°C of the organic acid is a value measured by neutralization titration. Examples of the organic acid having a pKa value at 25°C of 4.0 or greater include the organic acids described in Chemical Handbook (Basics), 4th Revised Edition, II-317 to II-321, Maruzen Publishing Co., Ltd (1993).

The content of the organic acid having a pKa value at 25°C of 4.0 or greater in the 1,6-hexanediol composition of the present invention may be any content at which the acid value of the composition of the present invention falls within the above-mentioned numerical value range; however, with regard to the 1,6-hexanediol composition of the present invention, the upper limit of the content of the organic acid having a pKa value at 25°C of 4.0 or greater is preferably 2000 ppm by mass or less, more preferably 1000 ppm by mass or less, and even more preferably 500 ppm by mass or less. The lower limit of the content is not particularly limited, and is preferably 0.001 ppm by mass or more, more preferably 0.01 ppm by mass or more, even more preferably 0.05 ppm by mass or more, and particularly preferably 0.1 ppm by mass or more. These upper limits and lower limits can be used in any combination.

When the 1,6-hexanediol composition of the present invention is derived from a biomass resource, the content of the organic acid having a pKa value at 25°C of 4.0 or greater in the 1,6-hexanediol composition is preferably 1 to 2000 ppm by mass, and more preferably 10 to 1000 ppm by mass, for the reason of economic efficiency in purification processes. The content of the organic acid having a pKa value at 25°C of 4.0 or greater in the 1,6-hexanediol composition of the present invention is preferably 0.001 to 2000 ppm by mass, more preferably 0.001 to 1000 ppm by mass, even more preferably 0.01 to 500 ppm by mass, and particularly preferably 0.1 to 500 ppm by mass. This tends to allow the effects of the present invention to be obtained more favorably.

Here, the content of the organic acid means the total content in a case where a plurality of organic acids are contained. The same also applies to the contents of other components. Furthermore, the content of the organic acid in the 1,6-hexanediol composition means the content of a free organic acid in principle; however, the content may include the content of an organic acid that has formed a salt.

When there is an organic acid having a pKa value at 25°C of less than 4.0 in the 1,6-hexanediol composition, such an organic acid is likely to be oxidized to form an aldehyde, and there is a risk that a polymer such as a polyester may be colored, or an odor may be generated. Furthermore, in 1,6-hexanediol derived from a biomass resource, amino acids are likely to remain as the organic acid having a pKa value of less than 4.0, and when these amino acids are present, there is a risk that a polymer such as a polyester or a polyurethane may be colored.

Therefore, in the 1,6-hexanediol composition of the present invention, the content of the organic acid having a pKa value at 25°C of less than 4.0 is preferably 2000 ppm by mass or less, more preferably 1000 ppm by mass or less, even more preferably 500 ppm by mass or less, and particularly preferably 0 ppm by mass (unblended).

In the present specification, the content of the organic acid is a value measured by inductively coupled plasma mass spectrometry (ICP-MS).

The organic acid having a pKa value at 25°C of less than 4.0 is not particularly limited as long as the pKa value at 25°C is less than 4.0, and examples thereof include amino acids such as glycine (pKa value 2.36), alanine (pKa value 2.30), valine (pKa value 2.26), leucine (pKa value 2.35), isoleucine (pKa value 2.21), serine (pKa value 2.13), cysteine (pKa value 1.88), methionine (pKa value 2.15), aspartic acid (pKa value 1.93), asparagine (pKa value 2.14), glutamic acid (pKa value 2.18), glutamine (pKa value 2.17), arginine (pKa value 2.05), lysine (pKa value 2.04), histidine (pKa value 1.70), phenylalanine (pKa value 2.26), tyrosine (pKa value 2.17), tryptophan (pKa value 2.35), and proline (pKa value 1.90). These may be used singly, or two or more kinds thereof may be used in combination.

When the organic acid having a pKa value at 25°C of less than 4.0 is used, it is desirable to use an organic acid that does not contain a nitrogen atom and has a pKa value as high as possible.

The 1,6-hexanediol composition of the present invention may be produced such that the total content of the alkali metal element falls within the above-described range. For example, since a 1,6-hexanediol composition as a commercially available product has a total content of an alkali metal element of less than 0.1 ppm by mass, for example, a metal salt of an acid such as acetic acid, phosphoric acid, or nitric acid and an alkali metal may be added to the 1,6-hexanediol composition as a commercially available product, and the total content of the alkali metal element may be adjusted to be within the above-described range. Similarly, since a 1,6-hexanediol composition as a commercially available product has an acid value of 0, an acid (preferably, an organic acid having a pKa value at 25°C of 4.0 or greater) may be added to the 1,6-hexanediol composition as a commercially available product, and the acid value may be adjusted to fall within the above-described range. Here, as the amount of the acid added is increased, the acid value also increases.

By adjusting the content of the alkali metal element and the acid value in the composition of the present invention to the above-described ranges, a polymer (for example, a polyester or a polyurethane having an ester bond) produced using the 1,6-hexanediol composition as a reaction raw material has satisfactory hydrolysis resistance. The reason why such an operating effect is exhibited is not clearly understood, but is speculated as follows.

Usually, when a polyester is heated in the presence of water, catalyst residues generated from a dehydration condensation reaction form coordinate bonds with the ester bond moieties of the polyester, causing a hydrolysis reaction of ester; however, the alkali metal element inhibits the catalyst residues from coordinating to the ester moiety of the polyester. This can favorably suppress the hydrolysis of a polyester and a polyurethane having an ester bond, and satisfactory hydrolysis resistance is obtained.

On the other hand, in a case where the composition contains a large amount of the alkali metal element, when a polymer such as a polyester is synthesized, there is a risk that the alkali metal element may form coordinate bonds with the catalyst and inhibit the inherent action of the catalyst, that is, the action of increasing the rate of the polymerization reaction. Thus, by incorporating only a specific amount of an alkali metal element into the 1,6-hexanediol composition of the present invention, excessive lowering of the inherent action of the catalyst due to the alkali metal element can be suppressed, and therefore, there is no adverse effect even when a polymer such as a polyester or a polyurethane is synthesized.

Therefore, since the 1,6-hexanediol composition of the present invention has a specific acid value and contains a specific amount of an alkali metal element, the composition can impart satisfactory hydrolysis resistance to the polymer, and at the same time, does not have adverse effect even when a polymer such as a polyester or a polyurethane is synthesized, and a polymer (for example, a polyester or a polyurethane having an ester bond) obtained by reacting the 1,6-hexanediol composition has satisfactory hydrolysis resistance.

<1,6-Hexanediol composition induced from biomass resource>

In recent years, environmental awareness has increased, and raw materials derived from biomass resources such as plants are desirable, rather than petroleum-derived raw materials that have an impact on global warming. Biomass resources are reusable organic resources produced from animals and plants, and preferred resources are plant resources such as wood, rice straw, rice husks, rice bran, long-stored rice, corn, sugar cane, cassava, soybeans, soybean pulp, bagasse, vegetable oils, oils and fats, waste paper, and papermaking residues. These biomass resources generally contain nitrogen element and many alkali metal elements and alkaline earth metal elements, such as sodium, potassium, magnesium, and calcium. Furthermore, methods for obtaining raw materials derived from biomass resources by utilizing microorganisms such as Corynebacteria and Escherichia coli from biomass resources, have also been disclosed, and in the present invention, for example, a 1,6-hexanediol composition induced from a biomass resource, such as a 1,6-hexanediol composition derived from a biomass resource obtained by the production method described in JP 2020-114227 A can be favorably used. When fermentation by microorganisms is utilized, usually, the hydrogen ion concentration (pH) of the fermentation liquid is adjusted using a neutralizing agent, in order to efficiently proceed with the fermentation. The neutralizing agent and the culture fluid contain large quantities of alkali metal elements and alkaline earth metals. Furthermore, it has been found, as a result of intensive investigation by the present inventors, that the 1,6-hexanediol derived from a biomass resource obtained by the production method described in JP 2020-114227 A is contaminated with organic acids such as acetic acid and succinic acid as impurities. These impurities are considered unnecessary and are removed by making full use of purification methods as far as cost allows. However, it has been found, as a result of intensive investigation of the present inventors, that organic acids and alkali metal elements are not unnecessary components to be present together with 1,6-hexanediol, and that co-presence thereof in specific amounts is rather an essential component for improving the hydrolysis resistance of a polymer such as a polyester. By combining known purification methods, a 1,6-hexanediol composition having a total content of alkali metal elements within the above-described range and an acid value within the above-described range, can be obtained.

<Polymer>

The polymer of the present invention is produced using the 1,6-hexanediol composition of the present invention as a reaction raw material.

The polymer is not particularly limited as long as it is a polymer having a structural unit derived from the 1,6-hexanediol composition of the present invention, and oligomers are also included therein. Examples thereof include a polyester, a polyurethane, a polycarbonate, a polyether, an epoxy-based polymer produced using the epoxy group-containing 1,6-hexanediol derivative as a raw material, and an acrylic polymer produced using the (meth)acryloyl group-containing 1,6-hexanediol derivative as a raw material. These may be used singly, or two or more kinds thereof may be used in combination.

Among them, for the reason that a hydrolysis resistance improving effect is more likely to be exhibited favorably, a polyester, a polyurethane, a polycarbonate, and a polyether are preferred, a polyester, a polyurethane, and a polyether are more preferred, and a polyester and a polyurethane are even more preferred.

Incidentally, the polymer of the present invention has at least a structural unit derived from the 1,6-hexanediol composition of the present invention, and also contains an alkali metal element and an organic acid derived from the 1,6-hexanediol composition. Here, when it is said in the present specification that the polymer of the present invention contains an organic acid, an aspect in which the polymer of the present invention contains a free organic acid, as well as an aspect in which the polymer has an organic acid-derived structural unit in the polymer skeleton are included. This is because an acid such as an organic acid may be incorporated into the polymer skeleton during polymerization.

The content of the structural unit derived from the 1,6-hexanediol composition of the present invention in 100% by mass of the polymer of the present invention is preferably 10% to 100% by mass, and more preferably 20% to 100% by mass. This tends to allow the effects to be obtained more favorably.

In the present specification, the content of each structural unit in the polymer is measured by NMR.

In the polymer of the present invention, the total content of the alkali metal elements (preferably, the total content of either or both of sodium metal element and potassium metal element) is preferably 0.05 to 500 ppm by mass, more preferably 0.05 to 400 ppm by mass, and even more preferably 0.05 to 300 ppm by mass. This tends to allow the effects of the present invention to be obtained more favorably.

The content of an organic acid having a pKa value at 25°C of 4.0 or greater in the polymer of the present invention is preferably 0.001 to 1000 ppm by mass, more preferably 0.001 to 500 ppm by mass, and even more preferably 0.05 to 250 ppm by mass. This tends to allow the effects to be obtained more favorably.

Here, as described above, when it is said in the present specification that the polymer of the present invention contains an acid, an aspect in which the polymer of the present invention contains a free organic acid, as well as an aspect in which the polymer of the present invention has an organic acid-derived structural unit in the polymer skeleton are included, and therefore, the content of the organic acid includes the content of free organic acid and the content of the organic acid-derived structural unit present in the polymer skeleton.

The content of an organic acid having a pKa value at 25°C of less than 4.0 in the polymer of the present invention is preferably 1000 ppm by mass or less, more preferably 500 ppm by mass or less, even more preferably 250 ppm by mass or less, and particularly preferably 0 ppm by mass (unblended). This can suppress coloring of the polymer.

The polymer may be modified. The modification is not particularly limited, and examples thereof include the modifications described in connection with the 1,6-hexanediol derivative. These may be used singly, or two or more kinds thereof may be used in combination. Among them, modification with (meth)acrylic acid is preferred. Particularly, in a case where the polymer is a polyester, it is preferable that the polymer is subjected to modification with (meth)acrylic acid.

The number average molecular weight (Mn) of the polymer of the present invention is preferably 500 to 1,000,000, and more preferably 3,000 to 500,000. **In** the present specification, the number average molecular weight (Mn) of the polymer is a value measured by gel permeation chromatography (GPC).

**In** the following description, the details of a polyester and a polyurethane among the above-described polymers will be described; however, the polymer is not limited to a polyester and a polyurethane.

<<Polyester>>

The polyester of the present invention is a compound produced using the 1,6-hexanediol composition of the present invention as a reaction raw material.

Specifically, the polyester of the present invention is a polycondensation product synthesized by subjecting a carboxylic acid and an alcohol to dehydration condensation to form ester bonds, and at least the 1,6-hexanediol composition of the present invention is used as the alcohol. Therefore, the polyester of the present invention is a reaction product (polycondensation product) obtained by a polycondensation reaction of a carboxylic acid and an alcohol, and the polyester of the present invention has at least a structural unit derived from the 1,6-hexanediol composition of the present invention, and also contains an alkali metal element or an organic acid derived from the 1,6-hexanediol composition.

The polyester of the present invention is preferably a polyester polyol.

Examples of the polyester polyol include a condensation-based polyester polyol and a lactone-based polyester polyol. The condensation-based polyester polyol is a reaction product of a low molecular weight polyhydric alcohol (a low molecular weight polyol such as ethylene glycol (EG), diethylene glycol, propylene glycol (PG), dipropylene glycol, (1,3- or 1,4-) butanediol, pentanediol, neopentyl glycol, hexanediol, cyclohexanedimethanol, glycerin, 1,1,1-trimethylolpropane (TMP), 1,2,5-hexanetriol, pentaerythritol, or 1,4-cyclohexanedimethanol; a sugar such as sorbitol; or the like) and a polyvalent basic carboxylic acid (glutaric acid, adipic acid, azelaic acid, fumaric acid, maleic acid, pimelic acid, suberic acid, sebacic acid, phthalic acid, terephthalic acid, isophthalic acid, dimer acid, pyromellitic acid, oligomer acid, hexahydrophthalic anhydride, 1,4-cyclohexanedicarboxylic acid, or the like). The lactone-based polyester polyol is, for example, a polycaprolactone polyol obtained by subjecting a lactone such as $\varepsilon$-caprolactone, $\alpha$-methyl-$\varepsilon$-caprolactone, or $\varepsilon$-methyl-$\varepsilon$-caprolactone to ring-opening polymerization.

Examples of the carboxylic acid include aliphatic polyvalent carboxylic acids such as succinic acid, adipic acid, azelaic acid, sebacic acid, glutaric acid, pimelic acid, suberic acid, dodecanedicarboxylic acid, maleic acid, and fumaric acid; alicyclic polyvalent carboxylic acids such as 1,3-cyclopentanedicarboxylic acid, 1,2-cyclohexanedicarboxylic acid, 1,3-cyclohexanedicarboxylic acid, 1,4-cyclohexanedicarboxylic acid, hexahydrophthalic anhydride, and 1,4-cyclohexanedicarboxylic acid; aromatic polyvalent carboxylic acids such as orthophthalic acid, isophthalic acid, terephthalic acid, naphthalenedicarboxylic acid, biphenyldicarboxylic acid, 1,2-bis(phenoxy)ethane-p,p'-dicarboxylic acid, and trimellitic acid; aliphatic monocarboxylic acids such as acetic acid, propionic acid, 2-ethylhexanoic acid, acrylic acid, and methacrylic acid; aromatic monocarboxylic acids such as benzoic acid, p-tertiary butylbenzoic acid, and p-hydroxybenzoic acid; various fatty acids derived from animal and vegetable oils, such as soybean oil fatty acids, tall oil fatty acids, linoleic acid, and eicosapentaenoic acid; and anhydrides thereof. These may be used singly, or two or more kinds thereof may be used in combination. Among them, aliphatic polyvalent carboxylic acids are preferred, and dodecanedicarboxylic acid is more preferred.

The alcohol that can be used in addition to the 1,6-hexanediol composition of the present invention is not particularly limited as long as it is a compound having a hydroxyl group (an alcoholic hydroxyl group or a phenolic hydroxyl group). Examples of the alcohol include aliphatic monoalcohols such as ethanol, butanol, and 2-ethylhexanol; aliphatic polyols such as ethylene glycol, neopentyl glycol, and trimethylolpropane; aromatic monohydric/polyhydric phenols such as phenol, cresol, and bisphenolA; and ethylene oxide extension products and hydrogenated alicyclic products thereof. These may be used singly, or two or more kinds thereof may be used in combination.

The content of the 1,6-hexanediol composition of the present invention in 100% by mass of the alcohol is preferably 10% to 100% by mass, and more preferably 50% to 100% by mass. This tends to allow the effects to be obtained more favorably.

The polyester of the present invention can be obtained by a known production method for polyesters. Specifically, the polyester can be synthesized by a production method of reacting the above-described carboxylic acid with the above-described alcohol at a reaction temperature of 150°C to 280°C while removing any produced water from the system. Furthermore, a reaction catalyst, an oxidation inhibitor, and the like may be used in combination during the synthesis.

The content of a structural unit derived from the 1,6-hexanediol composition of the present invention in 100% by mass of the polyester of the present invention is preferably 10% to 70% by mass, and more preferably 20% to 70% by mass. This tends to allow the effects to be obtained more favorably. **In** the present specification, the content of each structural unit in the polyester is measured by NMR.

The total content of the alkali metal element (preferably, total content of either or both of sodium metal element and potassium metal element) in the polyester of the present invention is preferably 0.05 to 500 ppm by mass, more preferably 0.05 to 400 ppm by mass, and even more preferably 0.05 to 300 ppm by mass. This tends to allow the effects to be obtained more favorably.

The content of an organic acid having a pKa value at 25°C of 4.0 or greater in the polyester of the present invention is preferably 0.001 to 1000 ppm by mass, more preferably 0.001 to 500 ppm by mass, and even more preferably 0.05 to 250 ppm by mass. This tends to allow the effects to be obtained more favorably.

The content of an organic acid having a pKa value at 25°C of less than 4.0 in the polyester of the present invention is preferably 1000 ppm by mass or less, more preferably 500 ppm by mass or less, even more preferably 250 ppm by mass or less, and particularly preferably 0 ppm by mass (unblended). This can suppress coloring. Here, as described above, when it is said in the present specification that the polymer of the present invention contains an acid, an aspect in which the polymer of the present invention contains a free organic acid, as well as an aspect in which the polymer has an organic acid-derived structural unit in the polymer skeleton are included, and therefore, the content of the organic acid includes the content of the free organic acid and the content of the organic acid-derived structural unit present in the polymer skeleton.

The number average molecular weight (Mn) of the polyester of the present invention is preferably 500 to 120,000, and more preferably 3,000 to 50,000. Incidentally, in the present specification, the number average molecular weight (Mn) of the polyester is a value measured by gel permeation chromatography (GPC).

«Polyurethane»

The polyurethane of the present invention is a reaction product obtained by reacting a polyol produced using the 1,6-hexanediol composition of the present invention as a reaction raw material, with a polyisocyanate. Furthermore, if necessary, a polyol produced without using a 1,6-hexanediol composition as a reaction raw material, a chain extending agent, a chain terminating agent, a crosslinking agent, and the like may be used in combination as reaction raw materials for the polyurethane of the present invention.

Therefore, the polyurethane of the present invention has a polyol-derived structural unit and a polyisocyanate-derived structural unit, and the polyurethane has at least a structural unit derived from the 1,6-hexanediol composition of the present invention and also optionally contains an alkali metal or an organic acid included in the 1,6-hexanediol composition of the present invention.

Examples of the polyol include polyols such as a polycarbonate polyol, a polyether polyol, and a polyester polyol. These polyols may or may not be produced using the 1,6-hexanediol composition as a reaction raw material, and it is desirable that the polyurethane of the present invention uses, at least as a reaction raw material, a polyol produced using the 1,6-hexanediol composition as a reaction raw material.

A polycarbonate polyol is a polyol obtained by a reaction between a glycol and a carbonate. Examples of the glycol include various saturated or unsaturated glycols such as the 1,6-hexanediol composition of the present invention, diethylene glycol, ethylene glycol, triethylene glycol, 1,2-propanediol, 1,3-propanediol, 1,3-butanediol, 1,4-butanediol, neopentyl glycol, pentanediol, 3-methyl-1,5-pentanediol, octanediol, 1,4-butanediol, dipropylene glycol, tripropylene glycol, polytetramethylene ether glycol, 2-methyl-1,3-propanediol, and 2-ethyl-2-butyl-1,3-propanediol; and alicyclic glycols such as 1,4-cyclohexane diglycol and 1,4-cyclohexane dimethanol.

Furthermore, examples of the carbonate include dialkyl carbonates (dimethyl carbonate, diethyl carbonate, and the like), ethylene carbonate, and diphenyl carbonate.

The polyether polyol is, for example, a polyether polyol obtained by subjecting an alkylene oxide to addition polymerization using various glycols as an initiator. Examples of the alkylene oxide include ethylene oxide, propylene oxide, butylene oxide, and tetrahydrofuran. Examples of the various glycols include glycols similar to the above-described glycol of polycarbonate diol.

As the polyol, in addition to those described above, polycaprolactone polyol, polyacrylic polyol, dimer diol, polybutadiene polyol, hydrogenated polybutadiene polyol, and the like can be used. These polyols may be used singly, or two or more kinds thereof may be used in combination.

As the chain extending agent, for example, aliphatic polyol compounds such as ethylene glycol, diethylene glycol, triethylene glycol, 1,2-propanediol, 1,3-propanediol, 1,3-butanediol, 1,4-butanediol, hexamethylene glycol, saccharose, methylene glycol, glycerin, sorbitol, and neopentyl glycol; aromatic polyol compounds such as bisphenol A, 4,4'-dihydroxydiphenyl, 4,4'-dihydroxydiphenyl ether, 4,4'-dihydroxydiphenyl sulfone, hydrogenated bisphenol A, and hydroquinone; water; and amine compounds such as ethylenediamine, 1,2-propanediamine, 1,6-hexamethylenediamine, piperazine, 2-methylpiperazine, 2,5-dimethylpiperazine, isophorone diamine, 4,4'-dicyclohexylmethanediamine, 3,3'-dimethyl-4,4'-dicyclohexylmethanediamine, 1,2-cyclohexanediamine, 1,4-cyclohexanediamine, aminoethylethanolamine, hydrazine, diethylenetriamine, triethylenetetramine, isophorone diamine, and 4,4'-methylenebis(2-chloroaniline), can be used. Regarding these chain extending agents, those derived from biomass resources can also be used. These chain extending agents may be used singly, or two or more kinds thereof may be used in combination. Among them, neopentyl glycol, 1,4-butanediol, trimethylolpropane, isophorone diamine, and 4,4'-methylenebis(2-chloroaniline) are more preferred.

As the polyol, it is particularly preferable to use a polyester polyol produced using the composition of the present invention as a raw material, and the content of the polyester polyol produced using the composition of the present invention as a raw material in 100% by mass of the polyol is preferably 10% to 100% by mass, and more preferably 50% to 100% by mass.

As the polyisocyanate, for example, aromatic polyisocyanates such as 1,3- and 1,4-phenylene diisocyanate, 1-methyl-2,4-phenylene diisocyanate, 1-methyl-2,6-phenylene diisocyanate, 1-methyl-2,5-phenylene diisocyanate, 1-methyl-2,6-phenylene diisocyanate, 1-methyl-3,5-phenylene diisocyanate, 1-ethyl-2,4-phenylene diisocyanate, 1-isopropyl-2,4-phenylene diisocyanate, 1,3-dimethyl-2,4-phenylene diisocyanate, 1,3-dimethyl-4,6-phenylene diisocyanate, 1,4-dimethyl-2,5-phenylene diisocyanate, diethylbenzene diisocyanate, diisopropylbenzene diisocyanate, 1-methyl-3,5-diethylbenzene diisocyanate, 3-methyl-1,5-diethylbenzene-2,4-diisocyanate, 1,3,5-triethylbenzene-2,4-diisocyanate, naphthalene-1,4-diisocyanate, naphthalene-1,5-diisocyanate, 1-methyl-naphthalene-1,5-diisocyanate, naphthalene-2,6-diisocyanate, naphthalene-2,7-diisocyanate, 1,1-dinaphthyl-2,2'-diisocyanate, biphenyl-2,4'-diisocyanate, biphenyl-4,4'-diisocyanate, 3,3'-dimethylbiphenyl-4,4'-diisocyanate, 4,4'-diphenylmethane diisocyanate, 2,2'-diphenylmethane diisocyanate, diphenylmethane-2,4-diisocyanate, and toluene diisocyanate; aliphatic polyisocyanates such as tetramethylene diisocyanate, 1,6-hexamethylene diisocyanate, dodecamethylene diisocyanate, and trimethylhexamethylene diisocyanate; alicyclic polyisocyanates such as 1,3-cyclopentylene diisocyanate, 1,3-cyclohexylene diisocyanate, 1,4-cyclohexylene diisocyanate, 1,3-di(isocyanatomethyl)cyclohexane, 1,4-di(isocyanatomethyl)cyclohexane, lysine diisocyanate, isophorone diisocyanate, 4,4'-dicyclohexylmethane diisocyanate, 2,4'-dicyclohexylmethane diisocyanate, 2,2'-dicyclohexylmethane diisocyanate, and 3,3'-dimethyl-4,4'-dicyclohexylmethane diisocyanate; and the like can be used. Regarding these polyisocyanates, those derived from biomass resources can also be used. These may be used singly, or two or more kinds thereof may be used in combination. Among them, aromatic polyisocyanates are preferred, and 4,4'-diphenylmethane diisocyanate and toluene diisocyanate are more preferred.

For the purpose of controlling the molecular weight of the obtained polyurethane, chain terminating agents having one active hydrogen group may be used as necessary. Examples of these chain terminating agents include aliphatic monohydroxy compounds having a hydroxyl group, such as methanol, ethanol, propanol, butanol, and hexanol; and aliphatic monoamines having an amino group, such as morpholine, diethylamine, dibutylamine, monoethanolamine, and diethanolamine. These may be used singly, or two or more kinds thereof may be used as a mixture.

For the purpose of increasing the heat resistance or strength of the obtained polyurethane, crosslinking agents having three or more active hydrogen groups or isocyanate groups can be used as necessary.

The polyurethane of the present invention can be obtained by a known production method for polyurethanes. Specifically, for example, a method for producing a polyurethane by preparing the above-mentioned polyol, the above-mentioned polyisocyanate, and the above-mentioned chain extending agent and allowing them to react, may be mentioned. It is preferable that such a reaction is carried out at, for example, a temperature of 50°C to 100°C for 3 to 10 hours. Furthermore, the reaction may be carried out in an organic solvent.

As the organic solvent, for example, ketone solvents such as N,N-dimethylformamide, N,N-dimethylacetamide, N-methyl-2-pyrrolidone, methyl ethyl ketone, methyl-n-propyl ketone, acetone, and methyl isobutyl ketone; ester solvents such as methyl formate, ethyl formate, propyl formate, methyl acetate, ethyl acetate, isopropyl acetate, n-butyl acetate, isobutyl acetate, and secondary butyl acetate; and alcohol solvents such as methanol, ethanol, isopropyl alcohol, and butanol, can be used. Furthermore, regarding these organic solvents, those induced from biomass resources may also be used. These organic solvents may be used singly, or two or more kinds thereof may be used in combination.

When the polyurethane is produced using, as a raw material, a polyester polyol produced using the 1,6-hexanediol composition of the present invention as a raw material, the content of the structural unit derived from the polyester polyol of the present invention in 100% by mass of the polyurethane is preferably 10% to 90% by mass, and more preferably 20% to 90% by mass. This tends to allow the effects to be obtained more favorably.

Furthermore, the content of a structural unit derived from the 1,6-hexanediol composition of the present invention in 100% by mass of the polyurethane of the present invention is preferably 1% to 63% by mass, and more preferably 2% to 63% by mass. This tends to allow the effects to be obtained more favorably.

In the present specification, the content of each structural unit in the polyurethane is measured by NMR.

The total content of an alkali metal element (preferably, total content of either or both of sodium metal element and potassium metal element) in the polyurethane of the present invention is preferably 0.05 to 500 ppm by mass, more preferably 0.05 to 400 ppm by mass, and even more preferably 0.05 to 300 ppm by mass.

The content of an organic acid having a pKa value at 25°C of 4.0 or greater in the polyurethane of the present invention is preferably 0.001 to 1000 ppm by mass, more preferably 0.001 to 500 ppm by mass, and even more preferably 0.05 to 250 ppm by mass. This tends to allow the effects to be obtained more favorably.

The content of an organic acid having a pKa value at 25°C of less than 4.0 in the polyurethane of the present invention is preferably 1000 ppm by mass or less, more preferably 500 ppm by mass or less, even more preferably 250 ppm by mass or less, and particularly preferably 0 ppm by mass (unblended). This can suppress coloring. Here, as described above, when it is said in the present specification that the polymer of the present invention contains an acid, an aspect in which the polymer of the present invention contains a free organic acid, as well as an aspect in which the polymer has an organic acid-derived structural unit in the polymer skeleton are included, and therefore, the content of the organic acid includes the content of the free organic acid and the content of the organic acid-derived structural unit present in the polymer skeleton.

The number average molecular weight (Mn) of the polyurethane of the present invention is preferably 5,000 to 1,000,000, and more preferably 10,000 to 500,000. This tends to allow the effects to be obtained more favorably. Incidentally, in the present specification, the number average molecular weight (Mn) of the polyurethane is a value measured by gel permeation chromatography (GPC).

The polymer of the present invention can be used for various use applications. Specifically, the polymer can be used for a variety of use applications such as artificial leather, synthetic leather, shoes, thermoplastic resins, foamed resins, thermosetting resins, paints, laminating adhesives, elastic fibers, urethane raw materials, automobile parts, sporting goods, vibration insulating materials, vibration damping materials, fiber treatment agents, and binders.

The polyester of the present invention can be used for various use applications. Specifically, the polyester can be used for a variety of use applications such as solvent-based, water-based, and powdered coating materials for metals, automobiles, woodworking, and plastics; modifying agents for packaging materials and containers, optical films, and molded articles; surface layers, intermediate layers, foamed layers, and adhesive layers of artificial leather and synthetic leather; adhesives such as laminating adhesives; shoes, thermoplastic resins, foamed resins, thermosetting resins, elastic fibers, polyurethane raw materials, automobile parts, and sporting goods.

The polyurethane of the present invention can be used for various use applications. Specifically, the polyurethane can be used for a variety of use applications such as surface layers, intermediate layers, foamed layers, and adhesive layers of artificial leather and synthetic leather; various coating agents such as paints, metal surface treatment agents, and film primers; binders for inkjet printing, inks, textile printing, and glass fiber bundling agents; shoes, thermoplastic resins, foamed resins, thermosetting resins, adhesives such as laminating adhesives, vibration insulating materials, vibration damping materials, automobile parts, sporting goods, and fiber treatment agents.

An epoxy-based polymer produced using the epoxy group-containing 1,6-hexanediol derivative as a raw material can be used for various use applications.

Specifically, the epoxy-based polymer can be used for a variety of use applications such as various coating agents such as paints and metal surface treatment agents; and molded products of matrix resins and the like for wind turbines.

An acrylic polymer produced using the (meth)acryloyl group-containing 1,6-hexanediol derivative as a raw material can be used for various use applications.

Specifically, the acrylic polymer can be used for a variety of use applications such as various coating agents such as paints, metal surface treatment agents, and film coatings; and binders for inks and UV inkjet printing.

Furthermore, the 1,6-hexanediol composition of the present invention can also be used for the modification of PBS resins, PET resins, PTT resins, PBT resins, and the like by adding the composition.

In the present specification, the notation "to" means values equal to or greater than the value before the notation "to", and equal to or less than the value after the notation "to". Furthermore, in the present specification, when a plurality of numerical value ranges are disclosed by the notation "to" for a certain characteristic or the like, the upper limit and the lower limit of each numerical value range can be used in any combination. For example, when two numerical value ranges of 0.001 to 500 ppm by mass and 0.05 to 250 ppm by mass are disclosed for the content of a certain compound, it is implied that numerical value ranges of 0.001 to 250 ppm by mass and 0.05 to 500 ppm by mass are also disclosed in addition to the ranges of 0.001 to 500 ppm by mass and 0.05 to 250 ppm by mass.

EXAMPLES

Hereinafter, the present invention will be described specifically by way of Examples and Comparative Examples; however, the present invention is not intended to be limited to these only.

(Example 1: Preparation of 1,6-hexanediol composition 1 (1,6-HD composition 1))

1,6-Hexanediol manufactured by Ube Industries, Ltd., which had an alkali metal element content of 0.02 ppm by mass of sodium as determined by ICP-MS analysis and an acid value of 0 mg KOH/g, was heated and melted at 80°C, potassium acetate was added thereto such that the potassium content would be 1.7 ppm by mass, 15 ppm by mass of acetic acid was further added thereto, and the mixture was cooled to obtain a 1,6-hexanediol composition 1. The acid value of the 1,6-hexanediol composition 1 was 0.02 mg KOH/g.

(Example 2: Preparation of 1,6-hexanediol composition 2 (1,6-HD composition 2))

1,6-Hexanediol manufactured by Ube Industries, Ltd., which had an alkali metal element content of 0.02 ppm by mass of sodium as determined by ICP-MS analysis and an acid value of 0 mg KOH/g, was heated and melted at 80°C, sodium acetate was added thereto such that the sodium content would be 995 ppm by mass, 400 ppm by mass of acetic acid was further added thereto, and the mixture was cooled to obtain a 1,6-hexanediol composition 2. The acid value of the 1,6-hexanediol composition 2 was 0.48 mg KOH/g.

(Example 3: Preparation of 1,6-hexanediol composition 3 (1,6-HD composition 3))

1,6-Hexanediol manufactured by Ube Industries, Ltd., which had an alkali metal element content of 0.02 ppm by mass of sodium as determined by ICP-MS analysis and an acid value of 0 mg KOH/g, was heated and melted at 80°C, potassium acetate was added thereto such that the potassium content would be 998 ppm by mass, 200 ppm by mass of succinic acid was further added thereto, and the mixture was cooled to obtain a 1,6-hexanediol composition 3. The acid value of the 1,6-hexanediol composition 3 was 0.11 mg KOH/g.

(Production Example 1) (Preparation of genetically modified Escherichia coli)

A plasmid A was prepared, in which glycerol dehydratase $\alpha$, $\beta$, and $\gamma$ subunit genes derived from Citrobacter freundii, and a dehydratase reactivation factor gene were introduced between BamHI and HindIII restriction enzyme cleavage sites of pACYC184 (NIPPON GENE CO., LTD.).

A plasmid B was prepared, in which Escherichia coli-derived HpaI gene as 4,6-dihydroxy-2-oxo-hexanoate aldolase, Escherichia coli-derived HpcG gene as 4,6-dihydroxy-2-oxo-hexanoate 4-dehydratase, Arabidopsis thaliana-derived NADPH-dependent oxidoreductase 2-alkenal reductase (GenBank: CAC01710.1) gene as 6-hydroxy-3,4-dehydro-2-oxohexanoate 3-reductase, PanE gene of Lactococcus lactis as 6-hydroxy-2-oxohexanoate 2-reductase, and Clostridium difficile-derived HadA gene as 2,6-dihydroxy-hexanoate CoA-transferase and 6-hydroxyhexanoyl-CoA-transferase were introduced between BamHI and EcoRI restriction enzyme cleavage sites of the multiple cloning site (MCS) of pUC19 (NIPPON GENE CO., LTD.).

A plasmid C was prepared, in which Clostridium difficile-derived HadB and HadC genes as $\alpha$ and $\beta$ subunit genes of 2-hydroxyisocaproyl-CoA dehydratase, Clostridium difficile-derived HadI gene as 2-hydroxyisocaproyl-CoA dehydratase activating enzyme, Treponema denticola-derived trans-2-enoyl-CoA reductase (GenBank: AE017248) gene as 6-hydroxy-2,3-dehydro-hexanoyl-CoA 2,3-reductase, Nocardia iowensis-derived carboxylic acid reductase (GenBank: AAR91681.1) gene as 6-hydroxyhexanoate 1-reductase, and Rhodococcus-derived 6-hydroxyhexanoate dehydrogenase (GenBank: AAN37489.1) gene as 6-hydroxyhexanal 1-reductase were introduced into pCOLADuet-1 (Novagen, Inc.) such that HadB, HadC, and HadI genes were introduced into MCS1 and the other genes were introduced into MCS2.

In the present section, the term gene refers to an open reading frame containing a stop codon encoding each enzyme, and each gene is introduced into a plasmid such that a sequence containing a T7 promoter and a ribosome binding site, is present upstream of the gene, and a sequence containing a T7 terminator is present downstream of the gene. Each gene can be expressed in a large amount in a host strain of Escherichia coli, in which T7 RNA polymerase is expressed by appropriate induction of expression.

Plasmids A, B, and C were sequentially transformed in chemically competent cells of BL21 Star (DE3) (Invitrogen Corporation) by a heat shock method, and the transformants were selected on an LB plate containing appropriate antibiotics. As a result, a BL21 Star (DE3) strain containing all the plasmids A, B, and C was obtained. In this way, an Escherichia coli having genes encoding ten enzymes of the 1,6-hexanediol pathway consisting of 4,6-dihydroxy-2-oxo-hexanoate aldolase (2A in the drawings of JP 6680671 B2), 4,6-dihydroxy-2-oxo-hexanoate 4-dehydratase (2B in the drawings of JP 6680671 B2), 6-hydroxy-3,4-dehydro-2-oxohexanoate 3-reductase (2C in the drawings of JP 6680671 B2), 6-hydroxy-2-oxohexanoate 2-reductase (2D in the drawings of JP 6680671 B2), 2,6-dihydroxy-hexanoate CoA-transferase (2E in the drawings of JP 6680671 B2), 2,6-dihydroxy-hexanoyl-CoA 2-dehydratase (2F in the drawings of JP 6680671 B2), 6-hydroxy-2,3-dehydro-hexanoyl-CoA 2,3-reductase (2G in the drawings of JP 6680671 B2), 6-hydroxyhexanoyl-CoA-transferase (4F3 in the drawings of JP 6680671 B2), 6-hydroxyhexanoate 1-reductase (5R in the drawings of JP 6680671 B2), and 6-hydroxyhexanal 1-reductase (5S in the drawings of JP 6680671 B2), was prepared.

(Example 4: Preparation of 1,6-hexanediol composition 4 (1,6-HD composition 4))

The Escherichia coli was inoculated into an autoclave-sterilized medium (carbon sources: glucose, glycerin; nitrogen source: enzyme extract; inorganic salts: potassium phosphate, potassium hydroxide, vitamin B12; antibiotics: carbenicillin, kanamycin, chloramphenicol; pH: 7.0, among the above-described components, glucose and glycerin were raw materials derived from biomass resources), and cultured at 30°C for 2 to 3 hours under aerobic conditions. Thereafter, when the optical density at 600 nm of the Escherichia coli reached 0.3 to 0.6, isopropyl-$\beta$-thiogalactopyranoside was added to a final concentration of 0.5 mM, iron(II) sulfate was added to a final concentration of 10 $\mu$M, culturing was further carried out at 30°C for 3 hours, and the enzymes of the 1,6-HD pathway were expressed. After the expression, an appropriate amount of carbon sources (glucose and glycerin) were added thereto, and the inside of the culture vessel was placed under a nitrogen atmosphere to create anaerobic conditions. Under these conditions, culture was carried out at 30°C for 48 hours, and 1,6-hexanediol was produced. The culture fluid was centrifuged at 4°C for 20 minutes, the supernatant was collected and filtered using an appropriate membrane filter having a pore size of 0.2 to 0.4 μm, and a 1,6-hexanediol composition was obtained as a filtrate.

[Purification of 1,6-hexanediol composition]

<Step (a): Ion exchange to remove cations>

The cations included in the 1,6-hexanediol composition were removed. In step (a), cation exchange was carried out in a batch manner. The temperature for bringing into contact with a cation exchange resin was set to 40°C, DIAION SK1BH manufactured by Mitsubishi Chemical Corporation was added as the cation exchange resin into the 1,6-hexanediol composition, followed by stirring for 3 hours. After the stirring, filtration was performed, and a 1,6-hexanediol composition A was obtained as a filtrate.

<Step (b): Ion exchange to remove anions>

The anions included in the 1,6-hexanediol composition A were removed. In step (b), anion exchange was carried out in a batch manner. The temperature for bringing into contact with an anion exchange resin was set to 40°C, DIAION SA10AOH manufactured by Mitsubishi Chemical Corporation was added as the anion exchange resin into the 1,6-hexanediol composition, followed by stirring for 3 hours. After the stirring, filtration was performed, and a 1,6-hexanediol composition B was obtained as a filtrate.

<Step (c): Step of removing water>

Moisture included in the 1,6-hexanediol composition was removed. A thin film type distiller was used as the apparatus for step (c). The jacket temperature was set to 70°C, the 1,6-hexanediol-containing composition was continuously introduced, and water was distilled out from the top part. Simultaneously with the distillation of water, dehydrated 1,6-hexanediol composition C was continuously withdrawn from the bottom part as a bottom product. The moisture concentration in this 1,6-hexanediol composition C was 0.020% by mass (200 ppm by mass).

<Step (d): Distillation separation of low boiling point components>

Components included in the 1,6-hexanediol composition C and having a boiling point lower than that of 1,6-hexanediol were removed in a continuous distillation column. As the distillation column in step (d), an Oldershaw distillation column was used. The 1,6-hexanediol composition C obtained in step (c) was continuously fed into the distillation column, and the column top temperature was controlled at a constant temperature of 240°C. Continuous distillation was carried out from the top part of the column, continuous withdrawal was carried out from the bottom of the column, removal of low boiling point components in the 1,6-hexanediol composition C was carried out, and a 1,6-hexanediol composition D from which components having a lower boiling point than that of 1,6-hexanediol had been removed was withdrawn from the bottom of the column.

<Step (e): Distillation separation of high boiling point components>

Components included in the 1,6-hexanediol composition D and having a boiling point higher than that of 1,6-hexanediol were removed in a continuous distillation column. As the distillation column in step (e), an Oldershaw distillation column was used. The 1,6-hexanediol composition D obtained in step (d) was continuously fed into the distillation column, and the column bottom temperature was controlled to be constant at 260°C. Continuous withdrawal was carried out from the bottom of the column, and removal of high boiling point components in the 1,6-hexanediol composition D was carried out. A 1,6-hexanediol composition E from which components having a higher boiling point than that of 1,6-hexanediol had been removed was obtained from the top of the column (column top distillate). A 1,6-hexanediol composition 4 (1,6-HD composition 4) was prepared by adding potassium acetate to the 1,6-hexanediol composition E such that the potassium metal element concentration as determined by ICP-MS analysis would be 110 ppm by mass, and adding sodium acetate to the composition such that the sodium metal element concentration would be 190 ppm by mass.

(Example 5: Preparation of 1,6-hexanediol composition 5 (1,6-HD composition 5))

1,6-Hexanediol manufactured by Ube Industries, Ltd., which had an alkali metal element amount of 0.02 ppm by mass of sodium as determined by ICP-MS analysis and an acid value of 0 mg KOH/g, was heated and melted at 80°C, potassium acetate was added thereto such that the potassium content would be 500 ppm by mass, 1000 ppm by mass of acetic acid was further added thereto, and the mixture was cooled to obtain a 1,6-hexanediol composition 5. The acid value of the 1,6-hexanediol composition 5 was 0.95 mg KOH/g.

(Comparative Example 1: Preparation of 1,6-hexanediol composition 6 (1,6-HD composition 6))

1,6-Hexanediol manufactured by Ube Industries, Ltd., which had an alkali metal element amount of 0.02 ppm by mass of sodium as determined by ICP-MS analysis and an acid value of 0 mg KOH/g, was used as it was. The 1,6-hexanediol used had a purity of 99.93% as determined by gas chromatography analysis, and any peak other than that of 1,6-hexanediol was not observed.

(Comparative Example 2: Preparation of 1,6-hexanediol composition 7 (1,6-HD composition 7))

1,6-Hexanediol manufactured by Ube Industries, Ltd., which had an alkali metal element amount of 0.02 ppm by mass of sodium as determined by ICP-MS analysis and an acid value of 0 mg KOH/g, was heated and melted at 80°C, sodium hydroxide was added thereto such that the sodium content would be 997 ppm by mass, and the mixture was cooled to obtain a 1,6-hexanediol composition 7. The acid value of the 1,6-hexanediol composition 7 was 0 mg KOH/g.

(Comparative Example 3: Preparation of 1,6-hexanediol composition 8 (1,6-HD composition 8))

A 1,6-hexanediol composition 8 (1,6-HD composition 8) was obtained in the same manner as in Example 4, except that potassium acetate was added to the 1,6-hexanediol composition E of Example 4 such that the potassium metal element concentration would be 610 ppm by mass, and sodium acetate was added to the composition such that the sodium metal element concentration would be 190 ppm by mass.

(Comparative Example 4: Preparation of 1,6-hexanediol composition 9 (1,6-HD composition 9))

1,6-Hexanediol manufactured by Ube Industries, Ltd., which had an alkali metal element amount of 0.02 ppm by mass of sodium as determined by ICP-MS analysis and an acid value of 0 mg KOH/g, was heated and melted at 80°C, 200 ppm by mass of succinic acid was added thereto, and the mixture was cooled to obtain a 1,6-hexanediol composition 9. The acid value of the 1,6-hexanediol composition 9 was 0.11 mg KOH/g.

The analysis results of the 1,6-hexanediol compositions obtained in the Examples and Comparative Examples are shown in Tables 1 and 2.

Furthermore, in Tables 1 and 2, the detection limits of potassium metal element and sodium metal element were 0.003 ppm by mass and 0.003 ppm by mass, respectively.

[Table 1]

| Case | Example 1 | Example 2 | Example 3 | Example 4 | Example 5 |
|---|---|---|---|---|---|
| Sample | 1,6-HD composition 1 | 1,6-HD composition 2 | 1,6-HD composition 3 | 1,6-HD composition 4 | 1,6-HD composition 5 |
| Potassium metal element (ppm by mass) | 1.7 | 0 (detection limit or below) | 998 | 110 | 500 |
| Sodium metal element (ppm by mass) | 0.02 | 995 | 0.02 | 190 | 0.02 |
| Total content of alkali metal elements (ppm by mass) | 1.72 | 995 | 998.02 | 300 | 500.02 |
| Acid value (mg KOH/g) | 0.02 | 0.48 | 0.11 | 0.03 | 0.95 |

EP 4 559 892 A1

[Table 2]

| Case | Comparative Example 1 | Comparative Example 2 | Comparative Example 3 | Comparative Example 4 |
|---|---|---|---|---|
| Sample | 1,6-HD composition 6 | 1,6-HD composition 7 | 1,6-HD composition 8 | 1,6-HD composition 9 |
| Potassium metal element (ppm by mass) | 0 (detection limit or below) | 0 (detection limit or below) | 610 | 0 (detection limit or below) |
| Sodium metal element (ppm by mass) | 0.02 | 997 | 190 | 0.02 |
| Total content of alkali metal elements (ppm by mass) | 0.02 | 997 | 800 | 0.02 |
| Acid value (mg KOH/g) | 0 | 0 | 0 | 0.11 |

<Synthesis Example for polyester>

(Synthesis of polyester (A-1))

1048.5 parts by mass of the 1,6-hexanediol composition 1 and 2093.4 parts by mass of dodecanedioic acid (manufactured by INVISTA) were charged into a reaction vessel equipped with a stirring rod, a temperature sensor, and a rectification tube, and while allowing dry nitrogen to flow into the reaction vessel and stirring the compounds, a dehydration condensation reaction was carried out at normal pressure at 190°C to 210°C, while distilling off the generated water out of the system. At the time point when the acid value of the reaction product reached 30 or less, 16 mg of tetraisopropyl titanate was added thereto, and the reaction was continued while reducing the pressure to 200 to 100 mmHg. At the time point when the acid value reached 1.0, the degree of vacuum was gradually increased using a vacuum pump, and the reaction was completed. In this way, a polyester A-1 having a hydroxyl group value of 37.4 mg KOH/g, an acid value of 0.01 mg KOH/g, and a number average molecular weight (Mn) of 3000 was obtained.

(Synthesis of polyesters A-2 to A-9)

For the 1,6-HD compositions 2 to 9, polyesters A-2 to A-9 were synthesized by similar techniques. The reactions performed using the 1,6-HD compositions 7 and 8 did not proceed smoothly, and therefore, the reactions were discontinued.
The analysis results of the polyesters obtained in the above-described Examples and Comparative Examples are shown in Tables 3 and 4.

(Evaluation of hydrolysis resistance of polyester [hydrolysis resistance])

The hydrolysis resistance was evaluated by immersing the obtained polyesters in hot water at 100°C for 4 days, and determining the increase in the acid value caused by hydrolysis.

[Table 3]

| Case | Example 1 | Example 2 | Example 3 | Example 4 | Example 5 |
|---|---|---|---|---|---|
| Polyester Sample | Polyester A-1 | Polyester A-2 | Polyester A-3 | Polyester A-4 | Polyester A-5 |
| 1,6-HD used | 1,6-HD composition 1 | 1,6-HD composition 2 | 1,6-HD composition 3 | 1,6-HD composition 4 | 1,6-HD composition 5 |
| Number average molecular weight | 3,000 | 3,000 | 3,000 | 3,000 | 3,000 |
| Acid value before hot water treatment (mg KOH/g) | 0.01 | 0.02 | 0.01 | 0.01 | 0.02 |

14

(continued)

| Case | Example 1 | Example 2 | Example 3 | Example 4 | Example 5 |
|---|---|---|---|---|---|
| Acid value after hot water treatment (mg KOH/g) | 1.18 | 1.08 | 1.03 | 1.13 | 1.09 |

[Table 4]

| Case | Comparative Example 1 | Comparative Example 2 | Comparative Example 3 | Comparative Example 4 |
|---|---|---|---|---|
| Polyester Sample | Polyester A-6 | Polyester A-7 | Polyester A-8 | Polyester A-9 |
| 1,6-HD used | 1,6-HD composition 6 | 1,6-HD composition 7 | 1,6-HD composition 8 | 1,6-HD composition 9 |
| Number average molecular weight | 3,000 | Synthesis not possible | Synthesis not possible | 3,000 |
| Acid value before hot water treatment (mg KOH/g) | 0.01 | | | 0.01 |
| Acid value after hot water treatment (mg KOH/g) | 1.50 | | | 1.52 |

<Synthesis Example for polyurethane>

(Synthesis of polyurethane (A-3))

300.00 parts by mass (0.100 mol) of the polyester A-3 and 20.83 parts by mass (0.200 mol) of neopentyl glycol (NPG) were charged into a four-necked flask equipped with a thermometer, a stirrer, and an inert gas inlet port, 395.9 parts by mass of N,N-dimethylformamide (DMF) as a solvent was charged thereinto, and the compounds were dissolved. Next, 75.08 parts by mass (0.300 mol) of 4,4'-diphenylmethane diisocyanate (MDI) and 34.8 parts by mass (0.200 mol) of CORONATE T-80 were added thereto at 40°C, the mixture was allowed to react at 90°C until the NCO% reached 0.1% or less, and a DMF solution of a polyurethane (A-3) with a non-volatile content of 50.0% by mass was obtained. The proportions of A-3, NPG, and MDI are A-3 : NPG : MDI = 1 : 2 : 3.

(Synthesis of polyurethanes A-4, A-6, and A-9)

For the polyesters A-4, A-6, and A-9, polyurethanes A-4, A-6, and A-9 were synthesized by similar techniques.
The analysis results of the polyurethanes obtained in the above-described Examples and Comparative Examples are shown in Tables 5 and 6.

(Evaluation of hydrolysis resistance of polyurethane)

After blending the obtained polyurethane with a polyisocyanate crosslinking agent (solid content of polyurethane/solid content of crosslinking agent = 10/1 in mass ratio), immediately the mixture was preliminarily dried in a Werner Mathis at 70°C for 1 minute, and then drying was carried out at 120°C for 2 minutes to obtain a polyurethane coating film. This coating film was aged at 40°C for 2 days, and then the film physical properties (initial; breaking strength) were measured using an AUTOGRAPH (manufactured by SHIMADZU CORPORATION) in an environment at 23°C and 65% RH. Furthermore, as a hydrolysis resistance test, the same polyurethane coating film was left to stand for one week under the conditions of 70°C and 95%RH, moisture was further dried at normal temperature for one day, subsequently the film physical properties were measured in the same manner as for the initial state, and the degree of deterioration of the coating film was measured.
The hydrolysis resistance was evaluated according to the following criteria using the retention rate of the breaking strength from the initial value.
The results are shown in Tables 5 and 6.

A: The retention rate of the breaking strength is 90% or greater.

B: The retention rate of the breaking strength is 80% or greater and less than 90%.
C: The retention rate of the breaking strength is less than 80%.

[Table 5]

| Case | Example 3 | Example 4 |
|---|---|---|
| Polyurethane Sample | Polyurethane A-3 | Polyurethane A-4 |
| Polyester used | Polyester A-3 | Polyester A-4 |
| Initial breaking strength (MPa) | 71 | 70 |
| Breaking strength after wet heating (MPa) | 67 | 65 |
| Hydrolysis resistance | A | A |

[Table 6]

| Case | Comparative Example 1 | Comparative Example 4 |
|---|---|---|
| Polyurethane Sample | Polyurethane A-6 | Polyurethane A-9 |
| Polyester used | Polyester A-6 | Polyester A-9 |
| Initial breaking strength (MPa) | 71 | 72 |
| Breaking strength after wet heating (MPa) | 53 | 51 |
| Hydrolysis resistance | C | C |

From Tables 1 to 6, it was found that since the 1,6-hexanediol compositions of the present invention have an acid value of 0.001 to 1.0 mg KOH/g and a total content of alkali metal elements of 0.1 to 1000 ppm by mass, the polymers obtained by reacting the 1,6-hexanediol compositions (for example, polyesters and polyurethanes obtained by reacting the polyesters) have satisfactory hydrolysis resistance.

In the present specification, the number average molecular weight (Mn) of a polyester is a value measured by gel permeation chromatography (GPC) under the following conditions.

Measuring apparatus; HLC-8320GPC manufactured by Tosoh Corporation
Column; TSKgel 4000HXL, TSKgel 3000HXL, TSKgel 2000HXL, TSKgel 1000HXL manufactured by Tosoh Corporation were used by connecting them in series.
Detector; RI (differential refractometer)
Data processing; MULTISTATION GPC-8020 model II manufactured by Tosoh Corporation
Measurement conditions; Column temperature 40°C

Solvent tetrahydrofuran
Flow rate 0.1 ml/min

Standard; Monodisperse polystyrene
Sample; A 0.2% tetrahydrofuran solution calculated in terms of resin solid content, filtered through a microfilter (100 μl)
Standard sample: A calibration curve was created using the following standard polystyrenes.
(Standard polystyrenes)

"TSKgel standard polystyrene A-500" manufactured by Tosoh Corporation
"TSKgel standard polystyrene A-1000" manufactured by Tosoh Corporation
"TSKgel standard polystyrene A-2500" manufactured by Tosoh Corporation
"TSKgel standard polystyrene A-5000" manufactured by Tosoh Corporation
"TSKgel standard polystyrene F-1" manufactured by Tosoh Corporation
"TSKgel standard polystyrene F-2" manufactured by Tosoh Corporation
"TSKgel standard polystyrene F-4" manufactured by Tosoh Corporation
"TSKgel standard polystyrene F-10" manufactured by Tosoh Corporation

"TSKgel standard polystyrene F-20" manufactured by Tosoh Corporation
"TSKgel standard polystyrene F-40" manufactured by Tosoh Corporation
"TSKgel standard polystyrene F-80" manufactured by Tosoh Corporation
"TSKgel standard polystyrene F-128" manufactured by Tosoh Corporation
"TSKgel standard polystyrene F-288" manufactured by Tosoh Corporation
"TSKgel standard polystyrene F-550" manufactured by Tosoh Corporation

Furthermore, in the present specification, the number average molecular weight (Mn) of a polyurethane is a value measured by gel permeation chromatography (GPC) under the following conditions.

Measuring apparatus: High speed GPC apparatus ("HLC-8220GPC" manufactured by Tosoh Corporation)
Column: The following columns manufactured by Tosoh Corporation were used by connecting them in series.

$$\text{"TSKgel G5000" (7.8 mm I.D.} \times \text{30 cm)} \times \text{1 unit}$$

$$\text{"TSKgel G4000" (7.8 mm I.D.} \times \text{30 cm)} \times \text{1 unit}$$

$$\text{"TSKgel G3000" (7.8 mm I.D.} \times \text{30 cm)} \times \text{1 unit}$$

$$\text{"TSKgel G2000" (7.8 mm I.D.} \times \text{30 cm)} \times \text{1 unit}$$

Detector: RI (differential refractometer)
Column temperature: 40°C
Eluent: Tetrahydrofuran (THF)
Flow rate: 1.0 ml/min
Injection volume: 100 $\mu$L (tetrahydrofuran solution having a sample concentration of 0.4% by mass)
Standard sample: The following standard polystyrenes were used to create a calibration curve.

(Standard polystyrenes)

"TSKgel standard polystyrene A-500" manufactured by Tosoh Corporation
"TSKgel standard polystyrene A-1000" manufactured by Tosoh Corporation
"TSKgel standard polystyrene A-2500" manufactured by Tosoh Corporation
"TSKgel standard polystyrene A-5000" manufactured by Tosoh Corporation
"TSKgel standard polystyrene F-1" manufactured by Tosoh Corporation
"TSKgel standard polystyrene F-2" manufactured by Tosoh Corporation
"TSKgel standard polystyrene F-4" manufactured by Tosoh Corporation
"TSKgel standard polystyrene F-10" manufactured by Tosoh Corporation
"TSKgel standard polystyrene F-20" manufactured by Tosoh Corporation
"TSKgel standard polystyrene F-40" manufactured by Tosoh Corporation
"TSKgel standard polystyrene F-80" manufactured by Tosoh Corporation
"TSKgel standard polystyrene F-128" manufactured by Tosoh Corporation
"TSKgel standard polystyrene F-288" manufactured by Tosoh Corporation
"TSKgel standard polystyrene F-550" manufactured by Tosoh Corporation

Claims

1.  A 1,6-hexanediol composition comprising:

either or both of 1,6-hexanediol and a 1,6-hexanediol derivative; and
an alkali metal element,
wherein a total content of the alkali metal element with respect to a total amount of the composition is in a range of 0.1 to 1000 ppm by mass, and
the composition has an acid value in a range of 0.001 to 1.0 mg KOH/g.

2. The 1,6-hexanediol composition according to claim 1, wherein the 1,6-hexanediol composition further comprises an organic acid, and the organic acid has a pKa value at 25°C of 4.0 or greater.

3. The 1,6-hexanediol composition according to claim 1 or 2, wherein the 1,6-hexanediol composition is induced from a biomass resource.

4. A polymer produced using the 1,6-hexanediol composition according to claim 1 or 2 as a reaction raw material.

5. The polymer according to claim 4, wherein the polymer is a polyester or a polyurethane.

# EP 4 559 892 A1

| A. | CLASSIFICATION OF SUBJECT MATTER |
|---|---|

*C07C 31/20*(2006.01)i; *C08G 18/42*(2006.01)i; *C08G 63/12*(2006.01)i; *C12P 7/18*(2006.01)i
FI:   C07C31/20 Z; C08G18/42; C08G63/12; C12P7/18

According to International Patent Classification (IPC) or to both national classification and IPC

| B. | FIELDS SEARCHED |
|---|---|

Minimum documentation searched (classification system followed by classification symbols)

   C07C31/00-31/44; C08G18/00-18/87; C08G63/00-63/91; C12P7/00-7/66

Documentation searched other than minimum documentation to the extent that such documents are included in the fields searched

   Published examined utility model applications of Japan 1922-1996
   Published unexamined utility model applications of Japan 1971-2023
   Registered utility model specifications of Japan 1996-2023
   Published registered utility model applications of Japan 1994-2023

Electronic data base consulted during the international search (name of data base and, where practicable, search terms used)

   CAplus/REGISTRY (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | JP 03-34946 A (UBE IND., LTD.) 14 February 1991 (1991-02-14) | 4-5 |
|   | claims, page 1, left column, line 18 to page 2, upper left column, line 7, page 3, upper left column, lines 12-18, page 3, upper right column, lines 4-8, examples | |
| Y | | 1-3 |
| Y | JP 2016-060705 A (UBE IND., LTD.) 25 April 2016 (2016-04-25) | 1-3 |
|   | claims, paragraphs [0009], [0044], [0049], fig. 1 | |
| X | JP 2008-247742 A (UBE IND., LTD.) 16 October 2008 (2008-10-16) | 4-5 |
|   | claims, paragraphs [0003], [0013]-[0015], examples | |
| Y | | 1-3 |
| X | JP 2001-316312 A (ASAHI KASEI CORP.) 13 November 2001 (2001-11-13) | 4-5 |
|   | paragraph [0004] | |
| A | | 1-3 |
| Y | WO 2010/103945 A1 (TORAY INDUSTRIES, INC.) 16 September 2010 (2010-09-16) | 1-3 |
|   | claims, paragraphs [0001]-[0011], [0014]-[0022], [0031], [0036] | |
| A | | 4-5 |

☑ Further documents are listed in the continuation of Box C.        ☑ See patent family annex.

| | | |
|---|---|---|
| * | Special categories of cited documents: | "T" later document published after the international filing date or priority date and not in conflict with the application but cited to understand the principle or theory underlying the invention |
| "A" | document defining the general state of the art which is not considered to be of particular relevance | |
| "E" | earlier application or patent but published on or after the international filing date | "X" document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | |
| "P" | document published prior to the international filing date but later than the priority date claimed | "&" document member of the same patent family |

| Date of the actual completion of the international search | Date of mailing of the international search report |
|---|---|
| **16 August 2023** | **12 September 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

19

## INTERNATIONAL SEARCH REPORT

International application No.

**PCT/JP2023/025825**

| C. | DOCUMENTS CONSIDERED TO BE RELEVANT | |
|---|---|---|
| Category* | Citation of document, with indication, where appropriate, of the relevant passages | Relevant to claim No. |
| Y | JP 2001-114881 A (TORAY INDUSTRIES, INC.) 24 April 2001 (2001-04-24) claims, paragraphs [0009], [0010], [0017]-[0035] | 1-3 |
| A | | 4-5 |

Form PCT/ISA/210 (second sheet) (January 2015)

## INTERNATIONAL SEARCH REPORT
### Information on patent family members

International application No.

**PCT/JP2023/025825**

| Patent document cited in search report | | | Publication date (day/month/year) | Patent family member(s) | Publication date (day/month/year) |
|---|---|---|---|---|---|
| JP | 03-34946 | A | 14 February 1991 | (Family: none) | |
| JP | 2016-060705 | A | 25 April 2016 | (Family: none) | |
| JP | 2008-247742 | A | 16 October 2008 | (Family: none) | |
| JP | 2001-316312 | A | 13 November 2001 | (Family: none) | |
| WO | 2010/103945 | A1 | 16 September 2010 | US 2011/0313102 A1 claims, paragraphs [0002]-[0014], [0018]-[0023], [0032], [0037], examples<br>EP 2407515 A1<br>CN 102282211 A<br>KR 10-2011-0139200 A | |
| JP | 2001-114881 | A | 24 April 2001 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010035579 A
- JP 2020114227 A

- JP 6680671 B

### Non-patent literature cited in the description

- Chemical Handbook (Basics). Maruzen Publishing Co., Ltd, 1993, vol. II-317-II-321